# EUROPEAN PATENT SPECIFICATION

(11) **EP 1 214 032 B1**
(45) Date of publication and mention of the grant of the patent: **09.05.2007**
(21) Application number: 00959316.1
(22) Date of filing: 23.08.2000
(51) Int. Cl.: A61F 13/15

(54) **REFASTENABLE ABSORBENT ARTICLE**
WIEDERVERSCHLIESSBARER ABSORBIERENDER ARTIKEL
ARTICLE ABSORBANT REUTILISABLE

(30) Priority: 23.08.1999 US 150327 P; 11.08.2000 US 637431
(43) Date of publication of application: 19.06.2002
(73) Proprietor: KIMBERLY-CLARK WORLDWIDE, INC., Neenah, Wisconsin 54956 (US)
(72) Inventor: FREIBURGER, Sarah Jane, Marie, Kaukauna, WI 54130 (US); BRUEMMER-PRESTLEY, Mary, Anne, Appleton, WI 54911 (US); BORDAIN, Nefetari, Edris, Tucker, Georgia 30084 (US); SCHMOKER, Suzanne, Marie, Oshkosh, WI 54904 (US); THORSON, Russell, Evan, Appleton, WI 54914 (US); RENARD, Keith, Joseph, Oshkosh, WI 54904 (US); FELL, David, Arthur, Neenah, WI 54956 (US)
(74) Representative: Davies, Christopher Robert
(86) International application number: PCT/US2000/023078
(87) International publication number: WO 2001/013845

(56) References cited:
- EP-A- 0 412 579
- EP-A- 0 450 541
- GB-A- 2 244 422
- GB-A- 2 294 865
- US-A- 4 906 243

## Description

This invention relates to a refastenable absorbent article exhibiting improved body fit. More specifically, this invention relates to a refastenable disposable absorbent article sized to have certain ratios between certain sections that improve body fit and reduce the likelihood of fluid leakage.

Today, various disposable absorbent articles exist which are designed to absorb and retain body fluid and/or excrement. Examples of such articles include incontinence products such as pants, briefs and undergarments, baby diapers, feminine care menstrual pants, training pants, etc. While most of these articles perform satisfactorily for their intended purpose, some are difficult to remove from the body of the wearer. Many do not have a refastenable mechanism that allows the article to be easily adjusted during use or be easily removed from the wearer's body before the product is permanently discarded while maintaining an underwear like fit. Some of today's commercially available products do not conform well to the human body and this poor fit increases the likelihood of fluid leakage while the product is being worn. Therefore, there remains a need for a refastenable, disposable absorbent article that will provide improved fit to the human body and reduce the likelihood of fluid leakage.

Now, a refastenable absorbent article has been invented which is sized to have predetermined ratios between certain sections so as to improve body fit and reduce the likelihood of fluid leakage.

### SUMMARY OF THE INVENTION

Briefly, this invention relates to a refastenable absorbent article according to claim 1.

The general object of this invention is to provide a refastenable absorbent article exhibiting improved body fit. A more specific object of this invention is to provide a refastenable disposable absorbent article sized to have certain ratios that improve body fit and reduce the likelihood of fluid leakage.

Another object of this invention is to provide a refastenable absorbent article that is easy to manufacture, is relatively inexpensive and is easy to use.

A further object of this invention is to provide a refastenable absorbent article that provides the wearer with a choice of how to apply and/or remove the absorbent article, i.e. pull the article up around the wearer's torso versus refasten the article around the wearer's torso.

Still another object of this invention is to provide a refastenable absorbent article that can easily be adjusted to conform to a wearer's body.

Other objects and advantages of the present invention will become more apparent to those skilled in the art in view of the following description and the accompanying drawings.

### BRIEF DESCRIPTION OF THE DRAWINGS

Fig. 1 is a top plane view of a refastenable absorbent article shown in a preassembled flat configuration.
Fig. 2 is a cross-sectional view of Figure 1 taken along line 2-2.
Fig. 3 is a cross-sectional view of Figure 1 taken along the line 3-3.
Fig. 4 is a cross-sectional view of Figure 1 taken along the fine 4-4.
Fig. 5 is a top view of an alternative absorbent assembly.
Fig. 6 is a cross-sectional view of Fig. 5 taken along line 6-6.
Fig. 7 is a perspective view of the "in use" configuration of the refastenable absorbent article shown in Figure 1 once the front and back portions are secured together.
Fig. 8 is a top view of the back portion of the absorbent article with the elastic removed for clarity and showing one way to measure a ratio A₁/A₂ for the second section for obtaining improved body fit.
Fig. 9 is a top view of the back portion and crotch portion of the absorbent article with the elastic removed for clarity and showing an alternative way to measure a ratio A₃/A₄ for the second section for obtaining improved body fit.

### DETAILED DESCRIPTION OF THE PREFERRED EMBODIMENTS

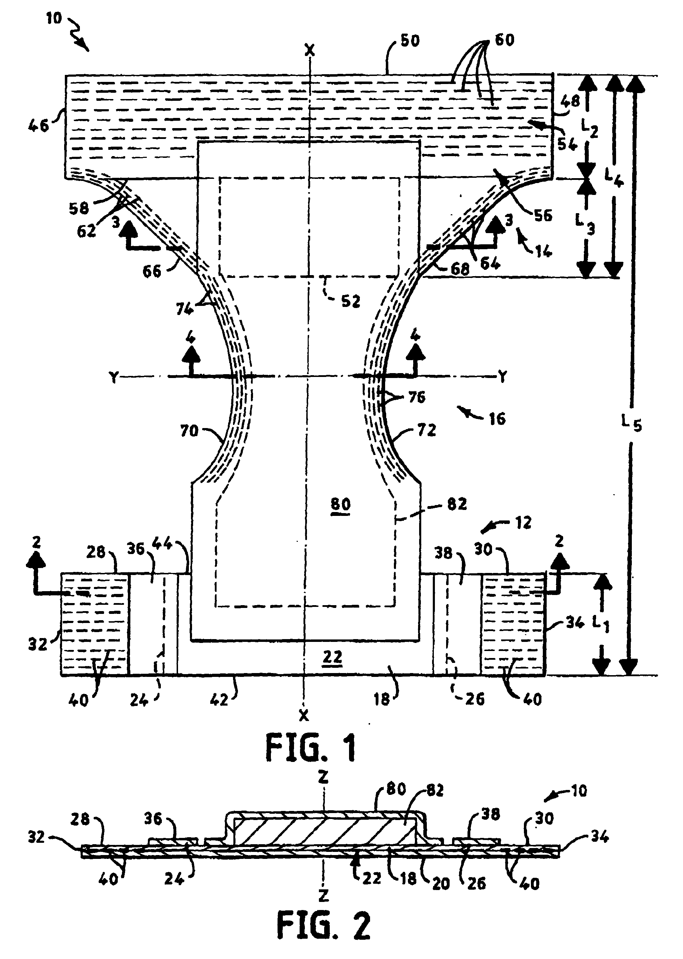

Referring to Figs. 1 and 2, a refastenable absorbent article 10 is shown. The absorbent article 10 is preferably disposable and has a longitudinal central axis X-X, a transverse central axis Y-Y and a vertical central axis Z-Z. The absorbent article 10 includes a front portion 12, a back portion 14 and a crotch portion 16. The crotch portion 16 connects the front portion 12 to the back portion 14. The front, back and crotch portions 12, 14 and 16 can be formed from a single piece of material or from two or more pieces of material. Each of the front, back and crotch portions 12, 14 and 16 can be a single layer of material or be formed as a laminate from two or more layers. As depicted, the front, back and crotch portions 12, 14 and 16 are shown as a laminate having an upper layer 18 and a lower layer 20. The upper layer, which is closest to the body of the wearer can be liquid permeable or liquid-impermeable. A thermoplastic film can be used to make the upper layer 18 liquid-impermeable. The lower layer 20 can also be liquid permeable or liquid-impermeable. Preferably, the lower layer 20 is liquid permeable and is formed from a soft material such as a non-woven. Spunbond is a non-woven material manufactured by Kimberly-Clark Corporation that is soft and pliable and functions well as the lower layer 20.

The front portion 12, which will contact the front of a wearer's torso in use, includes a central section 22 with first and second side edges 24 and 26, respectively. The front portion 12 also has first and second lateral sections 28 and 30, respectively, each of which are positioned adjacent to one of the first and second side edges 24 and 26. Each of the first and second lateral sections, 28 and 30 respectively, has an outer edge 32 and 34. Each of the first and second lateral sections 28 and 30 is releasably attached to the central section 22 by a fastener 36 and 38. Each of the fasteners 36 and 38 is depicted as a separate piece of material that joins each lateral section 28 and 30 to the central section 22. The fasteners 36 and 38 can be hook materials that releasably attach to the upper layer 18 that is formed as a loop material. Alternatively, the fasteners 36 and 38 can be loop materials that releasably attach to the upper layer 18 that is as a hook material. It is also possible to secure a separate piece of hook or loop material to the upper layer 18 to which the fasteners 36 and 38 will attach. The fasteners 36 and 38 can also include one or more frangible bonds, intermittent bonds, breakable bonds, or other similar type of structure. When such bonds are present, there will not be a need for a separate piece of material as is shown in Figs. 1 and 2. Likewise, other types of fasteners can also be employed, including glue, adhesives, co-adhesives, as well as mechanical fasteners, such as buttons and corresponding buttonholes, etc. Other fasteners are known to those skilled in the art.

It should be noted that the lateral sections 28 and 30 preferably contain elastic 40 to provide them with extendible properties. Alternatively, the lateral sections 28 and 30 can be formed from a material that exhibits elastomeric properties. When individual elastic members are utilized, the elastic 40 can be in the form of elongated strands, strips, bands, tape etc. and can have various configurations. The elastic 40 can be formed from rubber, polyurethane or other elastomeric materials. A suitable material is LYCRA® that is commercially available from the E.I. Du Pont De Nemours and Company. LYCRA® is a trademark of E.I. Du Pont De Nemours and Company having an office in Wilmington, Delaware.

The elastic 40 can include two or more members positioned between the upper and lower layers 18 and 20 and held in place by glue, an adhesive or some other type of binder. Preferably, the elastic 40 is in the form of multiple strands arranged in parallel rows relative to one another. Other arrangements for the elastic 40 can also be used.

Returning again to Figs. 1 and 2, the front portion 12 further has a first end 42 and a second end 44. A length L₁ represents the actual distance between the first end 42 and the second end 44. The length L₁ should be equal to or greater than about 2 inches (about 5 cm). Preferably, for an adult size garment, the length L₁ should range from between about 3 inches to about 7 inches (about 7.6 cm to about 18 cm). Most preferably, for an adult size garment, the length L₁ should range from between about 6 inches to about 7 inches (about 15 cm to about 18 cm). The significance of the dimension L₁ will be explained shortly.

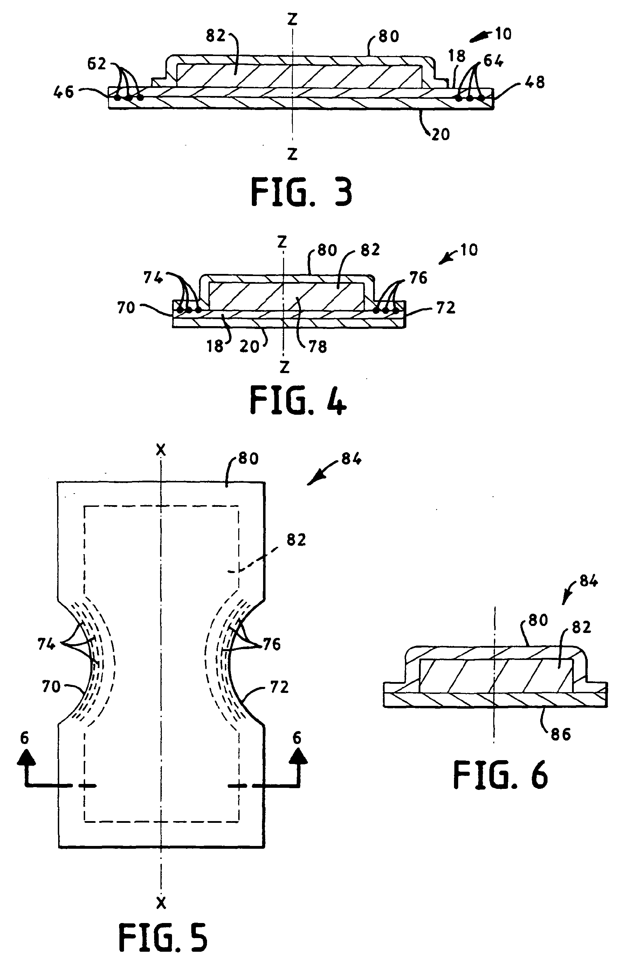

Referring to Figs. 1 and 3, the absorbent article 10 also includes the back portion 14 that will contact the back portion of a wearer's torso in use. The back portion 14 has first and second side edges 46 and 48, respectively, and first and second ends, 50 and 52 respectively. The back portion 14, as explained above, is depicted as a laminate having an upper layer 18 and a lower layer 20. The upper layer, which is closest to the body of the wearer can be liquid permeable or liquid-impermeable. Preferably, it is liquid permeable. The back portion 14 is divided into a first section 54 and a second section 56. The first section 54 has a length L₂ measured perpendicularly from the first end 50 to a line 58 drawn parallel thereto. The length L₂ should be equal to or greater than about 2 inches (about 5 cm). Preferably, for an adult size garment, the length L₂ should range from between about 3 inches to about 7 inches (about 7.6 cm to about 18 cm). Most preferably, for an adult size garment, the length L₂ should range from between about 6 inches to about 7 inches (about 15 cm to about 18 cm).

The first section 54 can be formed from an elastic material, an elastic composite or it can contain elastic 60. "Stretch bonded laminate" is an elastic material that can be used to form the first section 54. Stretch bonded laminate is manufactured by Kimberly-Clark Corporation having an office at 401 North Lake Street, Neenah, Wisconsin 54956. The elastic 60 can be in the form of elongated elastic strands, ribbons, bands, strips, etc. which extend transversely across the first section 54 from the first side edge 46 to the second side edge 48. Preferably, there are at least six elastic strands 60 contained in the first section 44. The number of elastic strands 60 can vary from two to about fifty. Preferably, there are from between about ten to about forty elastic strands 60. The elastic 60 functions to cause the back portion 14 of the absorbent article 10 to securely contact the back torso of the wearer and prevent fluid leakage from occurring at or about the waist opening.

The second section 56 of the back portion 14 has a length L₃ measured perpendicularly from the second end 52 to the line 58 drawn parallel thereto. The length L₃ should be equal to or greater than about 1 inch (about 2.5 cm). Preferably, for an adult size garment, the length L₃ should range from between about 3 inches to about 10 inches (about 8 cm to about 25 cm). Most preferably, for an adult size garment, the length L₃ should range from between about 4 inches to about 8 inches (about 10 cm to about 20 cm).

The second section 56 may or may not contain elastics 60 that extend transversely there across. By designing and manufacturing the absorbent article 10 to a ratio L₂/L₃, with a range of from between about 0.1 to about 2, one can improve the body fit of the absorbent article 10. Preferably, the ratio L₂/L₃ ranges from between about 0.2 to about 1.6, and more preferably, the ratio L₂/L₃ ranges from between about 0.3 to about 1.5. By manufacturing the back portion 14 to this desired ratio, one can improve the body fit of the disposable absorbent article 10 and minimize fluid leakage.

The second section 56 has first and second leg elastics 62 and 64 that are aligned adjacent to first and second edges 66 and 68. Preferably, the line 58 will intersect at a point where the first and second edges 66 and 68 meet the first and second edges 46 and 48, respectively. The leg elastics 62 and 64 can include one or more elastic strands, ribbons, bands or strips that are arranged approximately parallel to the edges 66 and 68 respectively. Preferably, there are from between about one to about six elongated elastic strands making up each leg elastic 62 and 64. Most preferably, each of the leg elastics 62 and 64 contains three elastic strands. The elastic strands can be aligned parallel to one another. For good results, the leg elastics 62 and 64 should be positioned inward about 1 inch (about 2.5 cm) from the first and second edges 66 and 68, respectively. More preferably, the leg elastics 62 and 64 should be positioned inward about 75 inches (about 2 cm) from the first and second edges 66 and 68, respectively. Most preferably, the leg elastics 62 and 64 should be positioned inward about .5 inches (about 1.3 cm) from the first and second edges 66 and 68.

The leg elastics 62 and 64 are shown extending from the side edges 46 and 48 of the first section 54 to the second end 52 of the back portion 14. One should note that the leg elastics 62 and 64 are completely contained within the second section 56 and do not extend into the first section 54. The leg elastics 62 and 64 function to gather the material from which the back portion 14 is formed at a location adjacent to the leg openings which are formed as the front and back portions 12 and 14 are secured together to form a pant-like article. Various types of elastics can be used to form the leg elastics 62 and 64. The leg elastics 62 and 64 should have a tension of from between about 10 grams to about 400 grams. Preferably, the leg elastics 62 and 64 should have a tension of from between about 50 grams to about 220 grams. More preferably, the leg elastics 62 and 64 should have a tension of from between about 80 grams to about 200 grams.

Referring to Figs. 1 and 4, the absorbent article 10 also includes the crotch portion 16. The crotch portion 16 connects the front portion 12 to the back portion 14. As noted above, the front portion, back portion and crotch portions can all be part of a single sheet of material. The crotch portion 16 has a first side edge 70 and a second side edge 72. The side edges 70 and 72 are arcuate in shape with the narrowest distance between the side edges 70 and 72 occurring along the transverse centerline Y-Y. The crotch portion 16 can be a single layer or a laminate as depicted. Since the crotch portion 16 acts as a baffle to body fluid that is absorbed by the absorbent article 10, it should be liquid-impermeable. If the crotch portion 16 is a laminate, at least one layer of the laminate should be liquid-impermeable. Polypropylene, polyethylene, or any other thermoplastic material works well as a liquid-impermeable layer. Preferably, the crotch portion 16 is a laminate formed from a layer of thermoplastic film and a layer of non-woven material. The non-woven material can be spunbond. Spunbond is a non-woven material manufactured and commercially sold by Kimberly-Clark Corporation having an office at 401 North Lake Street, Neenah, Wisconsin 54956.

The crotch portion 16 also includes first and second crotch elastics 74 and 76. The crotch elastics 74 and 76 are located adjacent to the first and second side edges 70 and 72, respectively. The crotch elastics 74 and 76 are preferably contoured to match the profile of the side edges 70 and 72. The crotch elastics 74 and 76 are located inward about 1 inch (about 2.5 cm) of the first and second side edges 70 and 72, respectively. Preferably, the crotch elastics 74 and 76 are located inward about .75 inches (about 2 cm) of the first and second side edges 70 and 72, respectively. More preferably, the crotch elastics 74 and 76 are located inward about .5 inches (about 1.3 cm) of the first and second side edges 70 and 72, respectively.

The crotch elastics 74 and 76 should have a tension of from between about 10 grams to about 400 grams. Preferably, the crotch elastics 74 and 76 should have a tension of from between about 50 grams to about 220 grams. More preferably, the crotch elastics 74 and 76 should have a tension of from between about 80 grams to about 200 grams.

The crotch elastics 74 and 76 can include one or more elongated elastic strands, ribbons or strips. Preferably, each of said first and second crotch elastics 74 and 76 includes at least two strands of elastics, and more preferably, at least three strands of elastics. The strands of each of the crotch elastics 74 and 76 can be aligned parallel to one another or they can be aligned in a non-parallel relationship, if so desired. A parallel alignment is preferred. The strands of each of the crotch elastics 74 and 76 can be spaced fairly close together, for example, within a millimeter of each other. The exact spacing can be adjusted depending on the size of the article, the width of the crotch portion 16, the strength of the crotch elastics 74 and 76, the material from which the article is constructed, etc.

The crotch elastics 74 and 76 can be positioned and secured between the layers 18 and 20 forming the laminate from which the crotch portion 16 is formed. Alternatively, the crotch elastics 74 and 76 can be adhered to an upper surface 78 of the upper layer 18 of the laminate, see Fig. 4. Preferably, the first and second crotch elastics 74 and 76 are bonded to the upper surface 78 of the upper layer 18 by an adhesive.

Referring to Figs. 1-4, the absorbent article 10 can include a liquid permeable liner 80. The liquid permeable liner 80 is also referred to as a bodyside cover. The liner 80 is not required but the absorbent article 10 will be described as including the liner 80. The liquid permeable liner 80, when present, can be formed from any natural or synthetic material that is liquid permeable. The liquid permeable liner 80 can also be formed from a non-woven material. Spunbond is a good material from which to construct the liquid permeable liner 80.

The liquid permeable liner 80 can be secured to at least one of the front, back or crotch portions 12, 14 or 16. The liquid permeable liner 80 is shown being directly attached to the crotch portion 16. Alternatively, the liquid permeable liner 80 can be indirectly attached to the crotch portion 16 via one or more layers. In this case, the intermediate layer would be secured to at least one of the front, back or crotch portions 12, 14 or 16. For example, the liquid permeable liner 80 can be secured to a liquid-impermeable baffle that is then adhesively secured to the crotch portion 16.

In Fig. 1, the periphery of the liquid permeable liner 80 and the periphery of the crotch portion 16 are shown to be coterminous. When the crotch elastics 74 and 76 are positioned on the upper surface 78 of the laminate, they can be adhesively secured to the liquid permeable liner 80 such that they are arranged adjacent to the side edges 70 and 72 of the crotch portion 16.

The absorbent article 10 further includes an absorbent 82 positioned below the liquid permeable liner 80. The absorbent 82 can be in direct contact with the upper layer 18 of the crotch portion 16. The absorbent 82 is designed to absorb body fluid, especially urine and can include one or more layers of absorbent material. The layers can be constructed of similar or different materials. Suitable materials for the absorbent 82 include cellulose, wood pulp fluff, rayon, cotton, and meltblown polymers such as polyester, polypropylene or coform. Coform is a meltblown air-formed combination of meltblown polymers, such as polypropylene, and absorbent staple fibers, such as cellulose. A preferred material is wood pulp fluff, for it is low in cost, relatively easy to form and has great absorbency. It should be noted that if two or more layers are utilized, that it is not necessary that all the layers be formed from the same material or have the same density.

The absorbent 82 can also be formed from a composite comprised of a hydrophilic material that can be formed from various natural or synthetic fibers, wood pulp fibers, regenerated cellulose or cotton fibers, or a blend of pulp and other fibers. A preferred material is airlaid tissue.

It is also possible and sometimes advantageous to insert a superabsorbent material into the absorbent 82 so as to increase its ability to absorb a large amount of fluid in relation to its own weight. Typical superabsorbents used in absorbent articles such as incontinence garments and diapers can absorb more than 10 times their weight in body fluid. The superabsorbent material can be inserted as particles, fibers or in sheet form. Hydroxyfunctional polymers have been found to be good superabsorbents for disposable absorbent articles. Such superabsorbents are commercially available from The Dow Chemical Company, Stockhausen, Inc., as well as other companies. Two such superabsorbents are DRYTECH® 2035 M and FAVOR® SXM 880. DRYTECH® is a registered trademark of The Dow Chemical Company having an office at 2030 Dow Center, Midland, Michigan 48642. FAVOR® is a registered trademark of Stockhausen, Inc. having an office at 2401 Doyle Street, Greensboro, North Carolina 27406. The superabsorbent can be a partially neutralized salt of cross-linked copolymers of polyacrylic acid. Other types of superabsorbent materials known to those skilled in the art can also be used.

Referring to Figs. 5 and 6, an absorbent assembly 84 is depicted which includes, from top to bottom, a liquid permeable bodyside liner 80, one or more layers of an absorbent 82 and a liquid-impermeable baffle 86. The liquid-impermeable baffle 86 can be formed from a thin layer of thermoplastic material such as polyethylene, polypropylene or other kinds of liquid-impermeable material. Preferably, the baffle 86 is formed from a liquid-impermeable film. It should be noted that the baffle could be formed from a laminate wherein at least one of the layers is liquid-impermeable.

In the absorbent assembly 84, the crotch elastics 74 and 76 are sandwiched between the liner 80 and the baffle 86. The crotch elastics 74 and 76 can be held in place by a glue, an adhesive, by ultrasonics, by heat, by pressure, by a combination of heat and pressure, or by some other bonding mechanism known to those skilled in the art. It is also possible to secure the crotch elastics 74 and 76 to the lower or exterior surface of the baffle 86 if desired. For good results, the crotch elastics 74 and 76 should be positioned within about 1 inch (about 2.5 cm) from the respective side edge 70 and 72. More preferably, the crotch elastics 74 and 76 should be positioned within about .75 inches (about 2 cm) from the respective side edge 70 and 72. Most preferably, the crotch elastics 74 and 76 should be positioned within about .5 inches (about 1.3 cm) from the respective side edge 70 and 72.

The absorbent assembly 84 can be placed or positioned on or over the crotch portion 16 of the absorbent article 10 and can be retained in place by an adhesive or some other type of fastener. Since the baffle 86 is liquid-impermeable, the crotch portion 16 would not have to be constructed from a liquid-impermeable material. The absorbent assembly 84 can be either permanently or releasably secured to at least one of the front, back or crotch portions 12, 14 or 16 of the absorbent article 10. Preferably, the absorbent assembly 84 is secured to the crotch portion 16 by an adhesive.

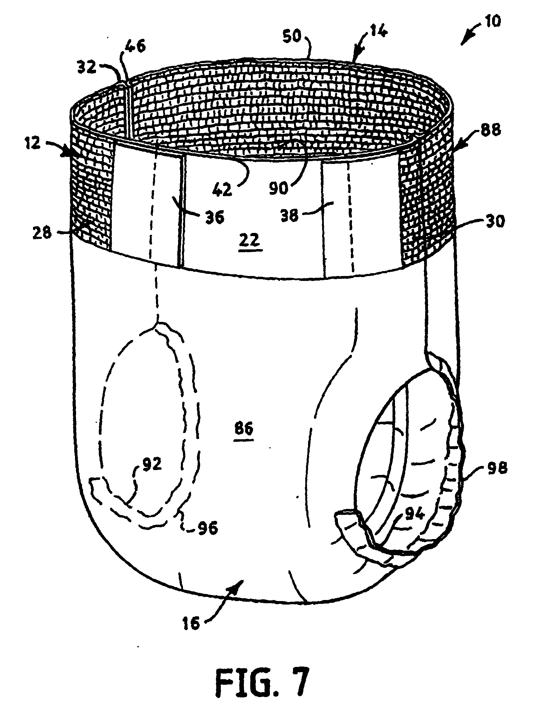

Referring now to Figs. 1 and 7, each of the outer edges 32 and 34 of the first and second lateral sections 28 and 30, are securely attached to one of the first and second side edges 46 and 48 to form a pant-like article 88. The pant-like article 88 has a waist opening 90 and a pair of first and second leg openings 92 and 94. The leg elastic 62, the crotch elastic 74, and possibly the elastic first section 54 can cooperate to form gathers 96 around the first leg opening 92. Likewise, the leg elastic 64, the crotch elastic 76, and possibly the elastic first section 54 can cooperate to form gathers 98 around the second leg opening 94. It should be noted that the absorbent article 10 could be sold in the assembled condition, as is shown in Fig. 7 or with the lateral regions detached from the central or front portions.

Referring again to Fig. 1, the back portion 14 of the absorbent article 10 has been described with reference to a first section 54 and a second section 56. The first section 54 has a length L₂ and the second section 56 has a length L₃, both measured parallel to the longitudinal central axis X-X. The combination of the length L₂ and the length L₃ equals a new length L₄. The length L₄ should range from between about 3 inches (about 7.5 cm) to about 25 inches (about 64 cm). Preferably, for an adult size garment, the length L₄ should range from between about 7 inches to about 17 inches (about 18 cm to about 43 cm). Most preferably, for an adult size garment, the length L₄ should range from between about 10 inches to about 15 inches (about 25 cm to about 38 cm).

In addition, the absorbent article 10 has an overall length L₅ measured perpendicularly from the first end 42 of the front portion 12 to the first end 50 of the back portion 14. The length L₅ should range from between about 10 inches (about 25 cm) to about 45 inches (about 114 cm). Preferably, for an adult size garment, the length L₅ should range from between about 20 inches (about 51 cm) to about 40 inches (about 102 cm). Most preferably, for an adult size garment, the length L₅ should range from between about 30 inches (about 76 cm) to about 35 inches (about 89 cm).

A ratio L₄/L₅ can be established which ranges from between about 0.1 to about 0.5. Preferably, the ratio L₄/L₅ ranges from between about 0.2 to about 0.5 and more preferably, the ratio L₄/L₅ ranges from between about 0.3 to about 0.4. By designing and manufacturing the absorbent article 10 to fit within the values established for the ratio L₄/L₅, one can be confident that the absorbent article 10 will exhibit improved body fit and be less likely to leak body fluid.

Returning again to the front portion 12 depicted in Fig. 1, it was mentioned that the front portion 12 had a length L₁. Since the front portion 12 is designed to mate with the back portion 14, it is advantageous to size the length L₁ to be approximately equal to the length L₂. This will provide an aesthetically pleasing pant-like article 88. However, if one desires, the length L₁ could be sized to be less than, equal to or greater than the length L₂.

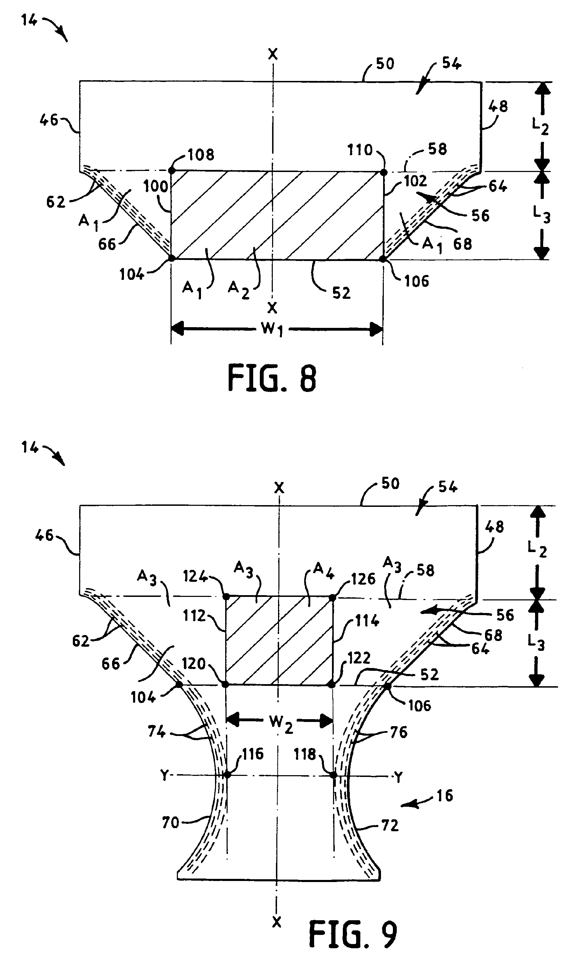

Referring now to Fig. 8, the back portion 14 of the absorbent article 10 is depicted with the elastic 60 removed for clarity of discussion only. The back portion 14, as described above, has a first section 54 and a second section 56. The first section 54 has a length L₂ that is equal to or greater than about 2 inches (about 5 cm) and the second section 56 has a length L₃ that is equal to or greater than about 1 inch (about 2.5 cm). The second section 56 includes a first area A₁ and a second area A₂. The first area A₁ is bounded by the second end 52, the line 58 drawn parallel to the first end 50, and the first and second side edges 66 and 68. In essence, the first area A₁ represents the total area of the second section 56. The second area A₂ is depicted as the cross hatched area bounded by first and second lines 100 and 102, the second end 52, and the line 58 drawn parallel to the first end 50. The first and second lines 100 and 102 are drawn perpendicular to the second end 52 at points 104 and 106 where the first and second side edges 66 and 68 intersect the second end 52. The first and second lines 100 and 102 extend vertically upward and intersect the line 58 at points 108 and 110, respectively. It has been found that if one divides the first area A₁ by the second area A₂, a ratio A₁/A₂ is established. If this ratio A₁/A₂ is maintained within a certain limited range, one can manufacture a disposable absorbent article 10 that exhibits improved body fit in the buttocks region of the wearer. Such improved body fit reduces the likelihood of fluid leakage from occurring which is of utmost importance to a consumer of such products.

The first area A₁ should be equal to or greater than about 175 cm². Preferably, for an adult size garment, the first area A₁ ranges from between about 400 cm² to about 1500 cm². More preferably, for an adult size garment, the first area A₁ ranges from between about 500 cm₂ to about 1200 cm². Most preferably, for an adult size garment, the first area A₁ ranges from between about 600 cm² to about 1100 cm². The second area A₂ should be equal to or greater than about 50 cm². The ratio A₁/A₂ should range from between about 1.3 to about 3.5. Preferably, the ratio A₁/A₂ should range from between about 1.7 to about 2.5, and more preferably, the ratio A₁/A₂ should range from between about 2.0 to about 2.3.

It should be noted that the first and second lines 100 and 102 are aligned parallel to one another and are spaced apart a distance denoted as W₁. W₁ represents the width of the second area A₂. The width W₁ should be equal to or greater than about 4 inches (about 10 cm). Preferably, the width W₁ should range from between about 5 inches (about 13 cm) to about 15 inches (about 38 cm), and more preferably, the width W₁ should range from between about 6 inches (about 15 cm) to about 11 inches (about 28 cm). The dimension W₁ can vary depending upon the type and size of absorbent article that is produced, as well as the age and physical size of the wearer. If the absorbent article is sized to be worn by an infant, the dimension W₁ will be less than if the absorbent article is designed to be worn by a toddler or by an adult. The gender of the wearer can also affect the dimension W₁.

Referring to Fig. 9, the back portion 14 and the crotch portion 16 of the absorbent article 10 are depicted with the elastic 60 removed from the back portion 14 for clarity of discussion only. The crotch portion 16 is joined at one end to the front portion 12, see Fig. 1. The crotch portion 16 has first and second side edges 70 and 72 and first and second crotch elastics 74 and 76 positioned adjacent to the first and second side edges 70 and 72. The back portion 14 is joined to the opposite end of the crotch portion 16, see Fig. 1. The back portion 14 has a first section 54 and a second section 56. The first section 54 has a length L₂ that is equal to or greater than about 2 inches (about 5 cm) and the second section 56 has a length L₃ that is equal to or greater than about 1 inch (about 2.5 cm). The second section 56 includes a first area A₃ and a second area A₄. The first area A₃ is bounded by the second end 52, the line 58 drawn parallel to the first end 50, and the first and second side edges 66 and 68. In essence, the first area A₃ represents the total area of the second section 56. The second area A₄ is depicted as the crosshatched area bounded by first and second lines 112 and 114, the second end 52, and the line 58 drawn parallel to the first end 50. The first and second lines 112 and 114 are drawn tangential to the first and second crotch elastics 74 and 76 at points 116 and 118 where the first and second crotch elastics are spaced closest together. Another way of stating this is that the first and second lines 112 and 114, respectively, are aligned tangentially to the inner most elastic strand of each of the first and second crotch elastics 74 and 76. For a symmetrically shaped article 10, this will occur on the transverse centerline Y-Y.

The first and second lines 112 and 114 extend vertically upward toward the first end 50 and intersect both the second end 52 at points 120 and 122, and the line 58 drawn parallel to the first end 50 at points 124 and 126. The crosshatched area bounded by the points 120, 122, 124 and 126 represent the second area A₄. It has been found that if one divides the first area A₃ by the second area A₄, that a ratio A₃/A₄ is established. If this ratio A₃/A₄ is maintained within a certain limited range, one can manufacture an absorbent article that exhibits improved body fit in the buttocks region of the wearer. Such improved body fit reduces the likelihood of fluid leakage from occurring which is of utmost importance to a disposable absorbent article.

The first area A₃ should be equal to or greater than about 175 cm². Preferably, the first area A₃ should range from between about 400 cm² to about 1500 cm². More preferably, the first area A₃ should range from between about 500 cm² to about 1200 cm², and most preferably, the first area A₃ ranges from between about 600 cm² to about 1100 cm². The second area A₄ should be equal to or greater than about 35 cm². The ratio A₃/A₄ ranges from between 2.5 to 4.0, and preferably, the ratio A₃/A₄ should range from between about 3.0 to about 3.7.

It should be noted that the first and second lines 112 and 114 are aligned parallel to one another and are spaced apart a distance denoted as W₂. W₂ represents the width of the second area A₄. The width W₂ should be equal to or greater than about 2 inches (about 5 cm). Preferably, the width W₂ should range from between about 3 inches (about 8 cm) to about 7 inches (about 18 cm), and more preferably, the width W₂ should range from between about 4 inches (about 10 cm) to about 6 inches (about 15 cm). The dimension W₂ can vary depending upon the type and size of absorbent article that is produced, as well as the age and physical size of the wearer. If the absorbent article is sized to be worn by an infant, the dimension W₂ will be less than if the absorbent article is designed to be worn by a toddler or by an adult. The gender of the wearer can also affect the dimension W₂.

While the invention has been described in conjunction with several specific embodiments, it is to be understood that many alternatives, modifications and variations will be apparent to those skilled in the art in light of the aforegoing description. Accordingly, this invention is intended to embrace all such alternatives, modifications and variations that fall within the scope of the appended claims.

## Claims

1. A refastenable absorbent article (10) comprising:
a) a front portion (12) having a central section (122) including first and second side edges (24, 26), first and second lateral sections (28, 30) each positioned adjacent to one of said first and second side edges and each of said first and second lateral sections being releasably attached to said central section, each of said lateral sections having an outer edge (32, 34), and said front portion having a first end and a second end (42, 44);
b) a crotch portion (16) joined at one end to said front portion, said crotch portion having first and second arcuate side edges (70, 72) and first and second crotch elastics (74, 76) positioned adjacent to said first and second side edges, respectively;
c) a back portion (14) joined to an opposite end of said crotch portion, said back portion having a first end (50), a second end (52) where the back portion joins the end of the crotch portion, and first and second side edges, said back portion being divided by a line (58) into a first section (54) and a second section (56), said second section having first and second leg elastics (62, 64) positioned adjacent to said first and second side edges, the leg elastics extending through the second section (56) of the back portion and not into the first section (54) thereof, said first section having a length L₂ measured perpendicularly from said first end of said back portion to said line (58) which is drawn parallel thereto, said length L₂ being equal to or greater than 2 inches (50 mm), and said second section having a length L₃ measured perpendicularly from said second end (52) to said line (58) drawn parallel to said first end, said length L₃ being equal to or greater than 1 inch (30 mm), said second section having a first side edge (66) and a second side edge (68) and a first area A₃ and a second area A₄, said first area A₃ representing an area bounded by said first and second side edges, said second end and said line drawn parallel to said first end, said second area A₄ representing an area bounded by first and second lines, said second end and said line drawn parallel to said first end, said first and second lines being drawn tangential to said first and second crotch elastics at points where said first and second crotch elastics are spaced closest together, said first and second lines intersecting said second end and said line drawn parallel to said first end, and
d) an absorbent (82) secured to at least one of said front, back or crotch portions, and each of said outer edges of said first and second lateral sections of said front portion being secured to one of said first and second side edges of said back portion to form a pant-like article having a waist opening (90) and a pair of leg openings (92, 94);
**characterized in that** said second section has a ratio A₃/A₄ ranging from between 2.5 to 4.0;.

2. The refastenable absorbent article of claim 1, further comprising a liquid permeable liner (80) secured to at least one of said front, back or crotch portions; wherein said absorbent is positioned below said liner.

3. The refastenable absorbent article of claim 1 or 2 wherein said ratio A₃/A₄ ranges from between 3.0 to 3.7.

4. The refastenable absorbent article of claim 1, said first section having multiple elastic strands (60) extending transversely across said first section from said first side edge to said second side edge, said first area being equal to or greater than 175 cm² and said second section having a ratio A₃/A₄ ranging from between 3.0 to 3.7; and further comprising a liquid permeable liner (80) secured to at least one of said front, back or crotch portions; wherein said absorbent is positioned below said liner.

5. The refastenable absorbent article of any of claims 1 to 3 wherein said first section contains elastic (60).

6. The refastenable absorbent article of any of claims 1 to 3 or 5 wherein A₃ is equal to or greater than 175 cm²:

7. The refastenable absorbent article of any preceding claim wherein A₃ ranges from between 400 cm² to 1500 cm².

8. The refastenable absorbent article of any preceding claim wherein A₃ ranges from between 500 cm² to 1200 cm².

9. The refastenable absorbent article of any preceding claim wherein said length L₃ ranges from between 3 inches (80 mm) to 10 inches (250 mm).

10. The refastenable absorbent article of any of claims 1 to 8 wherein said length L₃ ranges from between 4 inches (100 mm) to about 8 inches (200 mm).

11. The refastenable absorbent article of any preceding claim wherein said first and second lines are parallel to one another and the spacing between said first and second lines is greater than 2 inches (50 mm).

12. The refastenable absorbent article of any of claims 1 to 10 wherein said first and second lines are parallel to one another and the spacing between said first and second lines ranges from between 3 inches (80 mm) to 7 inches (180 mm).

13. The refastenable absorbent article of any of claims 1 to 10 wherein said first and second lines are parallel to one another and the spacing between said first and second lines ranges from between 4 inches (100 mm) to about 6 inches (150 mm).

## Patentansprüche

1. Wiederverschließbarer absorbierender Artikel (10) mit:
a) einem vorderen Bereich (12) mit einem mittleren Abschnitt (122), der einen ersten und einen zweiten Seitenrand (24, 26), einen ersten und einen zweiten seitlichen Abschnitt (28, 30) aufweist, wobei jeder angrenzend an den ersten oder den zweiten Seitenrand angeordnet ist und jeder erste und zweite seitliche Abschnitt lösbar an dem mittleren Abschnitt befestigt ist, wobei jeder der seitlichen Abschnitte einen äußeren Rand (32, 34) hat und der vordere Bereich ein erstes und ein zweites Ende (42, 44) hat;
b) einem Schrittbereich (16), der mit einem Ende des vorderen Bereiches verbunden ist, wobei der Schrittbereich einen ersten und einen zweiten bogenförmigen Seitenrand (70, 72) und einen ersten und einen zweiten Schrittgummizug (74, 76) hat, die angrenzend zum ersten bzw. zweiten Seitenrand angeordnet sind;
c) einem Rückenbereich (14), der mit einem gegenüberliegenden Ende des Schrittbereiches verbunden ist, wobei der Rückenbereich ein erstes Ende (50), ein zweites Ende (52), wo der Rückenbereich mit dem Ende des Schrittbereiches verbunden ist, und einen ersten und einen zweiten Seitenrand hat, wobei der Rückenbereich durch eine Linie (58) in einen ersten Abschnitt (54) und einen zweiten Abschnitt (56) geteilt ist, wobei der zweite Abschnitt einen ersten und einen zweiten Beingummizug (62, 64) hat, die angrenzend an den ersten und den zweiten Rand angeordnet sind, wobei sich die Beingummizüge durch den zweiten Abschnitt (56) des Rückenbereiches und nicht in dessen ersten Abschnitt (54) erstrecken, wobei der erste Abschnitt eine Länge L₂ hat, die rechtwinklig von dem ersten Ende des Rückenbereiches zu der Linie (58) gemessen wird, die dazu parallel gezogen wird, wobei die Länge L₂ gleich oder größer als 50 mm (2 Zoll) ist und der zweite Abschnitt eine Länge L₃ hat, die rechtwinklig von dem zweiten Ende (52) zu der Linie (58) gemessen wird, die parallel zu dem ersten Ende gezogen wird, wobei die Länge L₃ gleich oder größer als 30 mm (1 Zoll) ist, wobei der zweite Abschnitt einen ersten Seitenrand (66) und einen zweiten Seitenrand (68) und eine erste Fläche A₃ und eine zweite Fläche A₄ hat, wobei die erste Fläche A₃ eine Fläche darstellt, die durch den ersten und den zweiten Rand, das zweite Ende und die Linie begrenzt wird, die parallel zu dem ersten Ende gezogen wird, wobei die zweite Fläche A₄ eine Fläche darstellt, die von der ersten und der zweiten Linie, dem ersten Ende und der Linie begrenzt wird, die parallel zu dem ersten Ende gezogen wird, wobei die erste und die zweite Linie tangential zu dem ersten und dem zweiten Schrittgummizug an Punkten gezogen werden, wo der erste und der zweite Schrittgummizug am nächsten zueinander beabstandet sind, wobei die erste und die zweite Linie das erste Ende und die Linie schneiden, die parallel zu dem ersten Ende gezogen wird, und
d) einem Absorptionsmittel, das an wenigstens dem vorderen, Rücken- oder Schrittbereich befestigt ist, wobei jeder der äußeren Ränder des ersten und zweiten seitlichen Abschnitts des vorderen Bereiches an dem ersten oder dem zweiten Seitenrand des Rückenbereiches befestigt ist, um einen hosenähnlichen Artikel mit einer Taillenöffnung (90) und einem Paar Beinöffnungen (92, 94) zu bilden;
**dadurch gekennzeichnet, dass**
der zweite Abschnitt ein Verhältnis A₃/A₄ hat, das von 2,5 bis 4,0 reicht.

2. Wiederverschließbarer absorbierender Artikel nach Anspruch 1, ferner mit einer für Flüssigkeit durchlässigen Auskleidung (80), die an wenigstens dem vorderen, Rücken- oder Schrittbereich befestigt ist, wobei das Absorptionsmittel unter der Auskleidung angeordnet ist.

3. Wiederverschließbarer absorbierender Artikel nach Anspruch 1 oder 2, wobei das Verhältnis A₃/A₄ von 3,0 bis 3,7 reicht.

4. Wiederverschließbarer absorbierender Artikel nach Anspruch 1, wobei der erste Abschnitt mehrere Gummifäden (60) hat, die sich quer über den ersten Abschnitt von dem ersten Seitenrand zu dem zweiten Seitenrand erstrecken, wobei die erste Fläche gleich oder größer als 175 cm² ist und der zweite Abschnitt ein Verhältnis A₃/A₄ hat, das von 3,0 bis 3,7 reicht, und ferner eine für Flüssigkeit durchlässige Auskleidung (80) umfasst, die an wenigstens dem vorderen, Rücken- oder Schrittbereich befestigt ist, wobei das Absorptionsmittel unter der Auskleidung angeordnet ist.

5. Wiederverschließbarer absorbierender Artikel nach Anspruch 1 bis 3, wobei der erste Abschnitt Gummizug (60) enthält.

6. Wiederverschließbarer absorbierender Artikel nach Anspruch 1 bis 3 oder 5, wobei A₃ gleich oder größer als 175 cm² ist.

7. Wiederverschließbarer absorbierender Artikel nach einem der vorhergehenden Ansprüche, wobei A₃ von 400 cm² bis 1500 cm² reicht.

8. Wiederverschließbarer absorbierender Artikel nach einem der vorhergehenden Ansprüche, wobei A₃ von 500 cm² bis 1200 cm² reicht.

9. Wiederverschließbarer absorbierender Artikel nach einem der vorhergehenden Ansprüche, wobei die Länge L₃ von 80 mm (3 Zoll) bis 250 mm (10 Zoll) reicht.

10. Wiederverschließbarer absorbierender Artikel nach einem der Ansprüche 1 bis 8, wobei die Länge L₃ von 100 mm (4 Zoll) bis etwa 200 mm (8 Zoll) reicht.

11. Wiederverschließbarer absorbierender Artikel nach einem der vorhergehenden Ansprüche, wobei die erste und die zweite Linie parallel zueinander liegen und der Abstand zwischen der ersten und der zweiten Linie größer als 50 mm (2 Zoll) ist.

12. Wiederverschließbarer absorbierender Artikel nach einem der Ansprüche 1 bis 10, wobei die erste und die zweite Linie parallel zueinander liegen und der Abstand zwischen der ersten und der zweiten Linie von 80 mm (3 Zoll) bis 180 mm (7 Zoll) reicht.

13. Wiederverschließbarer absorbierender Artikel nach einem der Ansprüche 1 bis 10, wobei die erste und die zweite Linie parallel zueinander liegen und der Abstand zwischen der ersten und der zweiten Linie von 100 mm (4 Zoll) bis etwa 150 mm (6 Zoll) reicht.

## Revendications

1. Article absorbant réattachable (10) comprenant :
a) une portion frontale (12) ayant une section centrale (122) incluant des premier et second bords latéraux (24, 26), des première et seconde sections latérales (28, 30), dont chacune est positionnée adjacente à l'un desdits premier et second bords latéraux et chacune desdites première et seconde sections latérales étant réunie, de façon libérable, à ladite section centrale, chacune desdites sections latérales ayant un bord extérieur (32, 34) et ladite portion avant ayant une première extrémité et une seconde extrémité (42, 44) ;
b) une portion d'entrejambe (16) réunie, à une extrémité, à ladite portion frontale, ladite portion d'entrejambe ayant des premier et second bords latéraux arqués (70, 72) et des premier et second élastiques d'entrejambe (74, 76) positionnés adjacents, respectivement, auxdits premier et second bords latéraux;
c) une portion arrière (14) réunie à une extrémité opposée de ladite portion d'entrejambe, ladite portion arrière ayant une première extrémité (50), une seconde extrémité (52) où la portion arrière se réunit à l'extrémité de la portion d'entrejambe, et des premier et second bords latéraux, ladite portion arrière étant divisée par une ligne (58) en une première section (54) et une seconde section (56), ladite seconde section ayant des premier et second élastiques de jambe (62, 64) positionnés adjacents auxdits premier et second bords latéraux, les élastiques de jambe s'étendant au travers de la seconde section (56) de la portion arrière mais pas dans la première section (54) de celle-ci, ladite première section ayant une longueur L₂ mesurée perpendiculairement audit premier bord de ladite portion arrière jusqu'à ladite ligne (58) qui est tracée parallèlement à ladite première extrémité, ladite longueur L₂ étant égale ou supérieure à 2 pouces (50 mm), et ladite seconde section ayant une longueur L₃ mesurée perpendiculairement à partir de ladite seconde extrémité (52) jusqu'à ladite ligne (58) tracée parallèlement à ladite première extrémité, ladite longueur L₃ étant égale ou supérieure à 1 pouce (30 mm), ladite seconde section ayant un premier bord latéral (66) et un second bord latéral (68) et une première zone A₃ et une seconde zone A₄, ladite première zone A₃ représentant une zone limitée par lesdits premier et second bords latéraux, ladite seconde extrémité et ladite ligne tracée parallèlement à ladite première extrémité, ladite seconde zone A₄ représentant une zone limitée par des première et seconde lignes, ladite seconde extrémité et ladite ligne tracée parallèlement à ladite première extrémité, lesdites première et seconde lignes étant tracées tangentiellement auxdits premier et second élastiques d'entrejambe aux points où lesdits premier et second élastiques d'entrejambe sont le plus rapproché, lesdites première et seconde lignes étant sécantes à ladite seconde extrémité et à ladite ligne tracée parallèlement à ladite première extrémité, et
d) un absorbant (82) fixé à l'une au moins desdites portions avant, arrière ou d'entrejambe, et chacun desdits bords extérieurs desdites première et seconde sections latérales de ladite portion frontale étant fixé à l'un desdits premier et second bords latéraux de ladite portion arrière pour former un article semblable à une culotte ayant une ouverture de ceinture (90) et une paire d'ouvertures de jambe (92, 94) ;
**caractérisé en ce que** ladite seconde section a un rapport A₃/A₄ compris entre 2,5 et 4,0.

2. Article absorbant réattachable selon la revendication 1, comprenant en outre une doublure perméable aux liquides (80) fixée à l'une au moins desdites portions avant, arrière ou d'entrejambe, ledit absorbant étant positionné au-dessous de ladite doublure.

3. Article absorbant réattachable selon la revendication 1 ou 2, dans lequel ledit rapport A₃/A₄ est compris entre 3,0 et 3,7.

4. Article absorbant réattachable selon la revendication 1, dans lequel ladite première section comprend de multiples brins élastiques (60) s'étendant transversalement d'un côté à l'autre de ladite première section depuis ledit premier bord latéral jusqu'audit second bord latéral, ladite première zone étant égale ou supérieure à 175 cm² et ladite seconde section ayant un rapport A₃/A₄ compris entre 3,0 et 3,7 ; ledit article comprenant en outre une doublure perméable aux liquides (80) fixée à l'une au moins desdites portions avant, arrière ou d'entrejambe, ledit absorbant étant positionné sous ladite doublure.

5. Article absorbant réattachable selon l'une quelconque des revendications 1 à 3, dans lequel ladite première section contient de l'élastique (60).

6. Article absorbant réattachable selon l'une quelconque des revendications 1 à 3 ou 5, dans lequel A₃ est égal ou supérieur à 175 cm².

7. Article absorbant réattachable selon l'une quelconque des revendications précédentes, dans lequel A₃ est compris entre 400 cm² et 1500 cm².

8. Article absorbant réattachable selon l'une quelconque des revendications précédentes, dans lequel A₃ est compris entre 500 cm² et 1200 cm².

9. Article absorbant réattachable selon l'une quelconque des revendications précédentes, dans lequel ladite longueur L₃ est comprise entre 3 pouces (80 mm) et 10 pouces (250 mm).

10. Article absorbant réattachable selon l'une quelconque des revendications 1 à 8, dans lequel ladite longueur L₃ est comprise entre 4 pouces (100 mm) et environ 8 pouces (200 mm).

11. Article absorbant réattachable selon l'une quelconque des revendications précédentes, dans lequel lesdites première et seconde lignes sont parallèles l'une à l'autre et l'écartement entre lesdites première et seconde lignes est supérieur à 2 pouces (50 mm).

12. Article absorbant réattachable selon l'une quelconque des revendications 1 à 10, dans lequel lesdites première et seconde lignes sont parallèles l'une à l'autre et l'écartement entre lesdites première et seconde lignes est compris entre 3 pouces (80 mm) et 7 pouces (180 mm).

13. Article absorbant réattachable selon l'une quelconque des revendications 1 à 10, dans lequel lesdites première et seconde lignes sont parallèles l'une à l'autre et l'écartement entre lesdites première et seconde lignes est compris entre 4 pouces (100 mm) et environ 6 pouces (150 mm).
